# EUROPÄISCHE PATENTANMELDUNG

(11) **EP 2 135 575 A1**
(43) Veröffentlichungstag der Anmeldung: **23.12.2009**
(21) Anmeldenummer: 08010889.7
(22) Anmeldetag: 16.06.2008
(51) Int. Cl.: A61B 19/00

(54) **Instrumentenausrichtungsverfahren mit freier Referenz**

(71) Anmelder: BrainLAB AG, 85622 Feldkirchen (DE)
(72) Erfinder: Mueller, Matthias, 99084 Erfurt (DE); Gras, Florian, 07749 Jena (DE); Christian, Georg, 81545 München (DE); Uhde, Jörg, 80538 München (DE)
(74) Vertreter: Schwabe - Sandmair - Marx

(57) **Zusammenfassung**

Die Erfindung betrifft ein Instrumentenausrichtungsverfahren bei dem ein auszurichtendes medizinisches Instrument zu einem Zielpunkt (8) hin ausgerichtet wird, wobei die Raumlage des auszurichtenden Instruments mittels eines medizintechnischen Trackingsystems (13) bestimmt und verfolgt bzw. getrackt wird, wobei der Zielpunkt (8) durch ein frei bewegliches Zeigeinstrument (1), das ebenfalls mittels des medizintechnischen Trackingsystems (13) bestimmt und verfolgt wird, angezeigt wird, und dann das auszurichtende Instrument mit Hilfe einer mit dem Trackingsystem verbundenen medizintechnischen Navigation (15) auf den Zielpunkt (8) hin ausgerichtet wird.

## Beschreibung

Die Erfindung betrifft ein Instrumentenausrichtungsverfahren im medizinischen Umfeld. Insbesondere betrifft sie die Ausrichtung eines medizinischen Instruments zu einem Zielpunkt hin, wobei die Raumlage des auszurichtenden Instruments mittels eines medizintechnischen Trackingsystems bestimmt und verfolgt (getrackt) wird.

Grundsätzlich sind navigationsgestützte Ausrichtungsverfahren für Instrumente bekannt. Schon in der DE 196 39 615 A1 wird ein Navigationssystem beschrieben, bei dem registrierte Patientenbilddaten aus bildgebenden Verfahren (CT, MRI, PET,....) und getrackte Instrumente verwendet werden, um beispielsweise Eindring-Trajektorien von Instrumenten zu planen oder zusammen mit Patientenbilddaten auszugeben.

Ein Nachteil dieser chirurgischen Navigation und Planung liegt darin, dass zum Referenzieren und zum Aktualisieren der Patientenbilddaten-Zuordnung eine starr am Patienten bzw. Patientenkörperteil befestigte Referenzanordnung (Marker, Referenzstern) vorgesehen werden muss. Ferner ist eine solche "komplette" Navigation sehr aufwändig, speziell was die Vorab-Akquisition von Patientendaten betrifft. Außerdem ist schon die Anbringung von Navigations- bzw. Trackingreferenzen oftmals mit stark invasivem Vorgehen verbunden, und weil die Referenz in der Nähe der zu behandelnden Region angebracht werden muss, kann sie auch bei der Behandlung stören. Mit elastischen Bändern befestigte Referenzen können zu Ungenauigkeiten führen, und mechanische Hilfsmittel zum "Zielen" für medizinische Instrumente sind oftmals kompliziert zu handhaben und manchmal auch hinderlich während der Behandlung.

Es ist die Aufgabe der vorliegenden Erfindung, ein Instrumentenausrichtungsverfahren bereitzustellen, welches mindestens einen der oben genannten Nachteile des Standes der Technik überwindet. Insbesondere soll eine einfache, unkomplizierte bzw. unaufwändige Ausrichtungsmethode bereitgestellt werden, die dennoch eine hohe Genauigkeit aufweist.

Diese Aufgabe wird erfindungsgemäß durch ein Instrumentenausrichtungsverfahren gemäß dem Anspruch 1 gelöst. Die Unteransprüche definieren bevorzugte Ausführungsformen der Erfindung.

Beim Instrumentenausrichtungsverfahren der vorliegenden Erfindung wird der Zielpunkt durch ein frei bewegliches Zeigeinstrument, das ebenfalls mittels des medizinischen Trackingsystems bestimmt (identifiziert und positionsmäßig bestimmt) und verfolgt wird (d.h. getrackt wird), angezeigt und dann das auszurichtende Instrument mit Hilfe einer mit dem Trackingsystem verbundenen medizintechnischen Navigation auf den Zielpunkt hin ausgerichtet. Mit anderen Worten wird erfindungsgemäß von der komplizierten und aufwendigen "kompletten Navigation" Abstand genommen, und man macht sich die Erkenntnis zunutze, dass es in vielen Fällen einfach nur notwendig ist, einen Zielpunkt zu kennen, wobei die Ausrichtung des arbeitenden Instruments (auszurichtendes Instrument, Werkzeug) lediglich hin zu diesem Zielpunkt erfolgen muss, um ein befriedigendes Ergebnis erhalten zu können. Die Erfindung ersetzt also komplizierte Navigations- und Planungsschritte einfach durch das Anzeigen eines Zielpunkts. Ein solcher Zielpunkt kann aber mit einem Zeigerinstrument einfach und sehr genau (mit der Genauigkeit des Trackingsystems) angezeigt werden, so dass erfindungsgemäß eine unaufwändige aber genaue Instrumentenausrichtung stattfinden kann.

Man kann das erfindungsgemäße Verfahren und alle seine hier beschriebenen Ausführungsformen auch als ein medizintechnisches Planungsverfahren ansehen, dass es dem Behandelnden erlaubt, vor der eigentlichen Durchführung einer Maßnahme deren Erfolg zu überprüfen oder abzuschätzen, zum Beispiel die richtige Ausrichtung eines Instruments oder Werkzeugs und damit auch die richtige Ausrichtung des damit hergestellten Behandlungsergebnisses. Die vorliegende Erfindung dient nicht unmittelbar der Behandlung von Patienten sondern deren Vorbereitung und Erfolgssicherung durch die visuelle Unterstützung des Behandelnden. Die Erfindung kann auch als System- oder Vorrichtungserfindung aufgefasst werden, wobei die im Verfahren genutzten Vorrichtungen und ihre Kombinationen erfindungsgemäß umfasst sind und zusammengestellt werden.

Ein großer Vorteil und ein besonderer Aspekt der Erfindung liegt auch darin, dass das Zeigeinstrument eine Art "freie Referenz" bildet, die nicht mehr starr oder unverrückbar an dem Patientenkörperteil angebracht werden muss, weil es genügt, die Spitze des Zeigerinstruments am Zielpunkt orten zu können. Komplizierte und invasive Vorbereitungen oder die Anbringung einer starren Referenz an einem Patientenkörperteil sind damit nicht mehr notwendig.

Das auszurichtende Instrument kann bei einer Ausführungsform von einem Ansetzpunkt aus zum Zielpunkt hin ausgerichtet werden, wobei der Ansetzpunkt am Patientenkörperteil liegt und im Rahmen der anatomischen Gegebenheiten und der gewählten Behandlungsform frei durch den Behandelnden wählbar ist. Viele Ärzte können in einfacher Weise oder aus ihrer Erfahrung heraus sagen, wo ein Instrument angesetzt werden soll; dessen Bahn und Austrittspunkt zu bestimmen ist aber schwieriger, und hierbei hilft die vorliegende Erfindung.

Das Zeigeinstrument muss, wie oben schon ausgeführt, nicht fest und/oder starr über dem Patientenkörperteil, an dem Zielpunkt und/oder Ansatzpunkt liegen, angeordnet sein bzw. nicht fest oder starr mit dem Patientenkörperteil verbunden sein. Es ist vorzugsweise frei mit der Hand führbar bzw. frei handhabbar. Diese Begriffe sollen im Kontext der vorliegenden Offenbarung nicht alle Instrumente erfassen, die irgendwie oder mit großer Kraft oder mit großem Aufwand mit der Hand geführt werden können, sondern solche, deren freie Handhabung relativ einfach und der Natur des Instruments gemäß möglich ist. Speziell kann das Zeigeinstrument ein navigiertes Instrument mit einer Trackingreferenz und einem trackbaren Element sein, mit dem ein Zielpunkt angefahren werden kann. Dies trifft auf einen Pointer mit einer Pointerspitze zu, weil man hier weiß, wo das trackbare Element, nämlich die Spitze, im Abstand zu den Trackingreferenzen am Pointer liegt.

Das auszurichtende Instrument kann direkt durch das Tackingsystem getrackt werden, oder aber indirekt, indem beispielsweise eine Führung für das auszurichtende Instrument getrackt wird. Es kann eines oder mehrere der folgenden Instrumente sein bzw. umfassen:
- ein Meißel;
- ein chirurgischer Fräser (sog. "shaver");
- ein Schraubendreher;
- ein navigiertes Implantat, wie z.B. ein Nagel, eine Schraube oder Platte
- eine Punktionsnadel
- ein Trokar
- eine Drainage.

Im Kontext der vorliegenden Offenbarung werden medizintechnisch verwendete Vorrichtungen unter dem Begriff "Instrumente" zusammengefasst und die Erfindung lässt sich mit allen Instrumenten verwirklichen, bei denen es auf die Ausrichtung ankommt.

Gemäß einer Ausführungsform der vorliegenden Erfindung ist die medizintechnische Navigation eine bildfreie Navigation, also eine Navigation lediglich für die Instrumente ohne Patientenbilddaten oder irgendeine Referenz zu Patientenbilddaten. Im Rahmen dieser Navigation können zweidimensionale oder dreidimensionale Trajektorien Informationen, Winkelinformationen und/oder Abstandsinformationen in Bezug auf die Instrumente auf der Bildausgabe des Navigationssystems dargestellt werden. Eine andere Möglichkeit besteht in der Darstellung von Winkeln, Winkelabweichungen oder Abstandswerten zwischen Instrumententeilen, Instrumentenachsen oder zwischen geometrischen Eigenschaften, die den Instrumenten zugeordnet sind. Solche Darstellungen können in Zahlenform oder als Diagramme auf der Bildausgabe erfolgen, wobei speziell Balkendiagramme oder Kreis- bzw. Kreissegmentdiagramme verwendbar sind.

Als Referenzsystem für die medizintechnische Navigation bzw. deren bildliche Darstellung kann eines oder eine Kombination der folgenden Bezugssysteme gewählt werden:
- das Kamera-Bezugssystem des Trackingsystems;
- ein Referenzsystem, das durch eines der Instrumente definiert wird;
- ein Referenzsystem, das zumindest teilweise durch Verhältnisse zwischen zwei oder mehreren Instrumenten definiert wird.

Gemäß weiteren Aspekten betrifft die Erfindung ein Programm, das, wenn es auf einem Computer läuft oder in einem Computer geladen ist, den Computer veranlasst, ein Verfahren durchzuführen, wie es oben in verschiedenen Ausführungsformen erläutert wurde, sowie ein Computerprogramm-Speichermedium mit einem solchen Programm.

Einigen Behandlungen, die medizintechnisch unterstützt werden, liegt die Aufgabe zugrunde, auf einen bestimmten Punkt, den Zielpunkt, zu zielen bzw. ein Instrument dorthin auszurichten. Hier hilft die vorliegende Erfindung dabei, ein solches Ziel zu erreichen, wie dies in den folgenden, wiederum nur beispielhaften Ausführungsformen beschrieben wird. Das Ziel kann über eine definierte Zugangsbahn erreicht werden, die an einem definierten Ausgangspunkt oder Ansatzpunkt startet, oder über irgend eine andere Bahn, welche durch den Zielpunkt hindurch geht. Eine solche definierte Zugangsbahn könnte beispielsweise ein Bohrloch sein. Der Zielpunkt selbst könnte eine Struktur auf der Oberfläche oder unterhalb der Oberfläche eines Gelenkknorpels oder an einem Knochen in einer speziellen Region oder an einem Punkt sein, der durch ein Werkzeug oder ein Implantat definiert wird. Jedweder Ansatzpunkt oder Ausgangspunkt definiert dann eine Trajektorie zum Zielpunkt und navigierte Werkzeuge definieren beide Punkte, die durch eine chirurgische, nachfolgende Maßnahme miteinander in Relation gesetzt werden. Das Ziel kann dann durch eine definierte oder ausgewählte Trajektorie erreicht werden, wobei navigiertes Werkzeug und das Zeigegerät verwendet wird, welches auf den Zielpunkt deutet. Eine starr befestigte Referenz am Patientenkörperteil ist nicht notwendig. Mit einer bildlosen Navigationstechnik nach einer erfindungsgemäßen Ausführungsform wird es also möglich sein, eine Navigationsführung (Darstellung von Winkel- und Tiefeninformation) für ein navigiertes Werkzeug oder Implantat zu verwenden, ohne starre Referenzen anbringen zu müssen, was die navigierte Werkzeugverwendung und das Setzen eines Implantats beschleunigt.

Die Erfindung wird im Weiteren anhand von Ausführungsbeispielen und mit Hilfe der beiliegenden Zeichnungen näher erläutert. Sie kann alle hierin beschriebenen Merkmale einzeln sowie in jedweder sinnvollen Kombination aufweisen. In den Zeichnungen zeigen:
- Fig.1: eine schematische Darstellung eines erfindungsgemäßen Instrumentenausrichtungsverfahrens; und
- Fig. 2: schematische Darstellungen einer erfindungsgemäß unterstützenden Bildschirmausgabe.

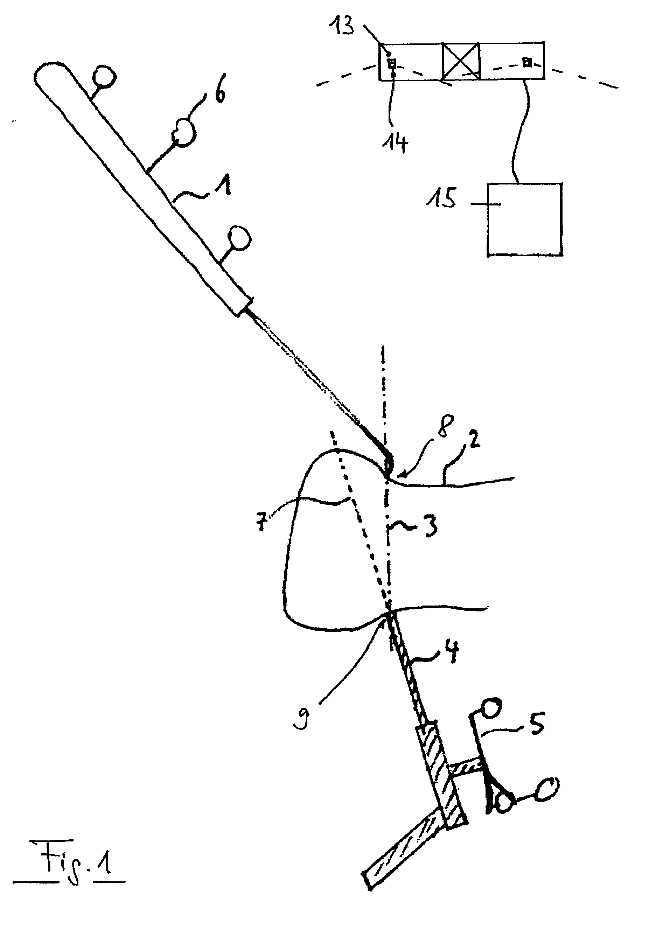

In der Fig. 1 ist ein Zeigeinstrument 1 dargestellt, nämlich ein Pointer mit Referenzmarkern am Griff, von denen einer mit dem Bezugszeichen 6 angedeutet ist. Der Pointer 1 hat eine Spitze, die mit 8 bezeichnet ist. Auf der unteren Seite ist eine Bohrerführung 4 dargestellt, die ebenfalls trackbar ist und hierzu die Trackingreferenz 5 aufweist. Sowohl Pointer 1 als auch die Bohrerführung 4 werden von dem nur schematisch dargestellten Trackingsystem 13 getrackt, das zwei Kameras aufweist, von denen eine das Bezugszeichen 14 trägt. Das Trackingsystem 13 ist integriert oder wird zusätzlich zu einem medizinischen Navigationssystem bereitgestellt, von dem - ebenfalls nur schematisch - die Bildausgabe 15 gezeigt ist. Mit dem Trackingsystem 13 kann die Raumlage des Pointers 1 und insbesondere auch seiner Spitze 8 festgestellt werden, ebenso wie die Raumlage der Bohrerführung 4.

Nur als Beispiel und repräsentativ für jedwedes Patientenkörperteil ist in der Fig. 1 ein Knochenende 2 gezeigt. Hier ist festzustellen, dass der Knochen zur Durchführung der vorliegenden erfindungsgemäßen Ausrichtung nicht unbedingt frei liegen muss; die Erfindung kann durchaus durchgeführt werden, ohne dass vorher ein invasiver Eingriff am Patientenkörperteil erfolgt ist.

Durch den Knochen 2 soll ein Bohrloch gebohrt werden, dessen Achse in Fig. 1 das Bezugszeichen 3 trägt. Die verlängerte Bohrerführungshülse ist ebenfalls als Achse eingezeichnet und hat in der Darstellung der Fig. 1 das Bezugszeichen 7 erhalten.

In der momentanen Ausrichtung, bei der die Bohrerführung 4 am Ansatzpunkt 9 des Knochens mit ihrer Spitze angesetzt worden ist, würde die Achse 7 und somit die Bohrlochrichtung nicht zum Zielpunkt führen, also zu dem Punkt, der durch die Pointerspitze 8 angezeigt wird. Wenn die Navigation nun genutzt wird, und zwar nur die Navigation der Instrumente ohne irgendwelche Daten für das Patientenkörperteil, kann durch eine Bewegung und Drehung der Bohrerführung 4 um den Ansatzpunkt 9 herum sichergestellt werden, dass die Achse 7 mit der angestrebten Bohrlochachse 3 übereinstimmt. Die operierende Person weiß aufgrund anatomischer Kenntnis, dass auf der Strecke zwischen Ansatzpunkt und Zielpunkt keine Strukturen liegen, die durch die Prozedur gefährdet oder dauerhaft geschädigt werden oder stellt dies vorher sicher. Deshalb werden auch keine Patientenkörperdaten für diese Navigation benötigt. Man muss lediglich den Ansatzpunkt wissen (Punkt 8, erhalten aus dem Tracking der Bohrerführung 4) und den Zielpunkt (Punkt 9, erhalten aus dem Tracking der Pointerspitze), und es lässt sich durch eine einfache Navigations-Darstellung planen, überprüfen und zeigen, ob die Bohrhülse in der richtigen Richtung liegt, so dass ein eingeführter Bohrer den Zielpunkt 8 erreichen kann.

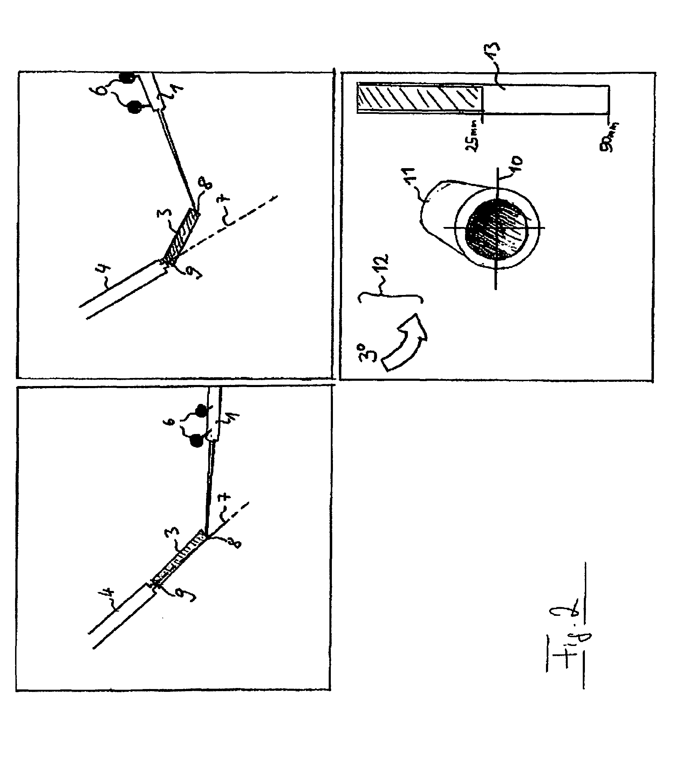

Anhand der Fig. 2 kann dies noch weiter erläutert werden. Die Fig. 2 zeigt drei Darstellungen, wie sie beispielsweise auf dem Navigationsbildschirm 15 zur Unterstützung des Behandelnden erscheinen würden. Man kann hier sehen, dass keinerlei Patientendaten dargestellt werden, sondern lediglich Bilder des navigierten Pointers 1 sowie der navigierten Bohrerführung 4. Zwischen den Spitzen der beiden Instrumente kann nun das angestrebte Bohrloch 3 als Hilfestellung eingeblendet werden, und in der linken oberen Darstellung sieht man, dass in einer Blickrichtung die Achse 7 schon fast mittig im virtuellen Bohrloch 3 liegt, so dass nur eine geringe Drehkorrektur in dieser Ebene notwendig ist. Das Bild rechts oben in Fig. 2 zeigt dann eine hierzu um 90° versetzte Ansicht, aus der hervorgeht, dass in dieser Ebene noch eine relativ große Drehung der Bohrerführung 4 notwendig ist, damit sie zwischen den Punkten 8 und 9 mittig in der virtuellen und angestrebten Bohrlochdarstellung zu liegen kommt.

Als Hilfe für den Verwender zur Ausrichtung des Werkzeugs kann dann beispielsweise eine Darstellung mit aufgezeichnet werden, wie sie rechts unten in Fig. 2 zu sehen ist. Dabei wird mit einem Fadenkreuz 10 die Mitte einer dreidimensionalen virtuellen rohrartigen Darstellung des Bohrlochs 11 angedeutet, wobei der nach hinten abstehende Fortsatz 11 des virtuellen "Bohrlochtunnels" immer weiter hinter dem vorderen Kreis verschwinden wird, je näher man der gewünschten Ausrichtung kommt. Winkelabweichungen sind durch Zahlenangaben und Richtungspfeile darstellbar wie oben links bei dem Bezugszeichen 12 gezeigt, und die Darstellung kann auch ein Balkendiagramm 13 enthalten, das beispielsweise den noch verbleibenden Abstand zum Zielpunkt bekannt gibt. Hierdurch erhält der Verwendende eine sehr eingehende und einfach verständliche bildliche Unterstützung und wird - ohne die Verwendung stark invasiver starrer Referenzanordnungen - die gewünschte Trajektorie des Bohrlochs mit hoher Genauigkeit auffinden und planen können.

Für die bildliche unterstützende Darstellung können, wie oben schon gesagt, unterschiedliche Referenzsysteme (Koordinatensysteme bzw. Bezugssysteme) verwendet werden, und wenn das Kamera-Bezugssystem verwendet wird, können beispielsweise absolute Richtungen wie "nach oben" oder "nach unten" angezeigt und verwendet werden, was eine sehr eingängige Bildunterstützung möglich macht. Referenzsysteme, die durch die Instrumente bzw. Implantate oder Werkzeuge bzw. deren räumliche Verhältnisse zueinander als Bezugssysteme bestimmt werden, sind vorteilhaft, wenn man verhindern will, dass sich die angestrebten Trajektorien auf dem Bildschirm bewegen. Solche Trajektorien (z.B. Bohrlochausrichtung oder Bohrerführungsausrichtung) könnten dann immer zentriert und parallel auf der Bildschirmoberfläche in einer Ansicht und senkrecht dazu in einer anderen Ansicht wiedergegeben werden.

Eine Kombination von Kamera-Bezugssystemen und Instrumenten-Relativ-Bezugssystemen könnte sich dann vorteilhaft auswirken, wenn eine sich nicht bewegende Trajektorie mit definierten Richtungen kombiniert werden soll. Es könnten auch Autopilot-Ansichten verwendet werden, um die Werkzeugführung zu unterstützen.

## Patentansprüche

1. Instrumentenausrichtungsverfahren bei dem ein auszurichtendes medizinisches Instrument zu einem Zielpunkt (8) hin ausgerichtet wird, wobei die Raumlage des auszurichtenden Instruments mittels eines medizintechnischen Trackingsystems (13) bestimmt und verfolgt bzw. getrackt wird, **dadurch gekennzeichnet, dass** der Zielpunkt (8) durch ein frei bewegliches Zeigeinstrument (1), das ebenfalls mittels des medizintechnischen Trackingsystems (13) bestimmt und verfolgt wird, angezeigt wird, und dann das auszurichtende Instrument mit Hilfe einer mit dem Trackingsystem verbundenen medizintechnischen Navigation (15) auf den Zielpunkt (8) hin ausgerichtet wird.

2. Instrumentenausrichtungsverfahren nach Anspruch 1, bei dem das auszurichtende Instrument von einem Ansetzpunkt (9) aus zum Zielpunkt (8) hin ausgerichtet wird.

3. lnstrumentenausrichtungsverfahren nach Anspruch 1 oder 2, bei dem das Zeigeinstrument (1) nicht fest und/oder starr gegenüber dem Patientenkörperteil, an dem Zielpunkt (8) und/oder Ansetzpunkt (9) liegen, angeordnet ist bzw. nicht fest oder starr mit dem Patientenkörperteil verbunden ist.

4. Instrumentenausrichtungsverfahren nach einem der Ansprüche 1 bis 3, bei dem das Zeigeinstrument (1) frei mit der Hand führbar bzw. frei handhabbar ist.

5. Instrumentenausrichtungsverfahren nach einem der Ansprüche 1 bis 4, bei dem das Zeigeinstrument (1) ein navigiertes Instrument mit einer Trackingreferenz (6) und einem trackbaren Element ist, mit dem ein Zielpunkt (8) angefahren werden kann, insbesondere ein Pointer (1) mit Pointerspitze ist.

6. Instrumentenausrichtungsverfahren nach einem der Ansprüche 1 bis 5, bei dem das auszurichtende Instrument direkt durch das Trackingsystem (13) getrackt wird.

7. Instrumentenausrichtungsverfahren nach einem der Ansprüche 1 bis 6, bei dem das auszurichtende Instrument indirekt durch das Trackingsystem (13) getrackt wird, insbesondere eine Führung (4) für das auszurichtende Instrument getrackt wird.

8. Instrumentenausrichtungsverfahren nach einem der Ansprüche 1 bis 7, bei dem das auszurichtende Instrument eines der folgenden Instrumente ist bzw. umfasst:
- ein medizintechnischer bzw. chirurgischer Bohrer;
- eine Führungshülse (4) für einen medizintechnischen bzw. chirurgischen Bohrer;
- ein Meißel;
- ein chirurgischer Fräser (sog. "shaver");
- ein Schraubendreher;
- ein navigiertes Implantat, wie z. B. ein Nagel eine Schraube oder eine Platte;
- eine Punktionsnadel;
- ein Trokar;
- eine Drainage.

9. Instrumentenausrichtungsverfahren nach einem der Ansprüche 1 bis 8, bei dem die medizintechnische Navigation eine bildfreie Navigation, also eine Navigation lediglich für die Instrumente ohne Patientenbilddaten oder irgendeine Referenz zu Patientenbilddaten ist.

10. Instrumentenausrichtungsverfahren nach einem der Ansprüche 1 bis 9, bei dem im Rahmen der medizintechnischen Navigation zwei- und/oder dreidimensionale Trajektorien-, Winkel- und/oder Abstandsinformationen in Bezug auf die Instrumente auf der Bildausgabe des Navigationssystems dargestellt werden.

11. Instrumentenausrichtungsverfahren nach einem der Ansprüche 1 bis 10, bei dem Winkel (12), Winkelabweichungen (11) oder Abstandswerte (13) zwischen Instrumententeilen, Instrumentenachsen, oder zwischen den Instrumenten zugeordneten geometrischen Eigenschaften, in Zahlenform oder als Diagramme, speziell Balkendiagramme, auf der Bildausgabe des Navigationssystems dargestellt werden.

12. Instrumentenausrichtungsverfahren nach einem der Ansprüche 1 bis 11, bei dem als Referenzsystem für die medizintechnische Navigation bzw. deren bildliche Darstellung eines oder eine Kombination der folgenden Bezugssysteme gewählt wird:
- das Kamera-Bezugssystem des Trackingsystems;
- ein Referenzsystem, das durch eines der Instrumente definiert wird;
- ein Referenzsystem, das zumindest teilweise durch Verhältnisse zwischen zwei oder mehreren Instrumenten definiert wird.

13. Programm, das, wenn es auf einem Computer läuft oder in einem Computer geladen ist, den Computer veranlasst, ein Verfahren gemäß einem der Ansprüche 1 bis 12 durchzuführen.

14. Computerprogramm-Speichermedium, das ein Programm nach Anspruch 13 aufweist.
